(19) Europäisches Patentamt
European Patent Office
Office européen des brevets

(11) **EP 0 617 595 B1**

(12) **EUROPÄISCHE PATENTSCHRIFT**

(45) Veröffentlichungstag und Bekanntmachung des Hinweises auf die Patenterteilung:
**12.03.1997 Patentblatt 1997/11**

(21) Anmeldenummer: **92924687.4**

(22) Anmeldetag: **08.12.1992**

(51) Int. Cl.$^6$: **A61F 2/30**, A61F 2/38

(86) Internationale Anmeldenummer:
**PCT/EP92/02832**

(87) Internationale Veröffentlichungsnummer:
**WO 93/11720 (24.06.1993 Gazette 1993/15)**

(54) **KÜNSTLICHES GELENK**

**ARTIFICIAL JOINT**

**ARTICULATION ARTIFICIELLE**

(84) Benannte Vertragsstaaten:
**AT BE CH DE DK ES FR GB GR IE IT LI LU MC NL PT SE**

(30) Priorität: **11.12.1991 DE 4140837**
**31.01.1992 DE 4202717**

(43) Veröffentlichungstag der Anmeldung:
**05.10.1994 Patentblatt 1994/40**

(60) Teilanmeldung: **96108124.7**
**96108125.4**

(73) Patentinhaber:
• **Theusner, Joachim, Dr.**
**80539 München (DE)**
• **Kubein-Meesenburg, Dietmar, Prof. Dr.**
**37547 Kreiensen (DE)**

• **NÄGERL, Hans Dr.**
**37130 Gleichen (DE)**

(72) Erfinder:
• **KUBEIN-MEESENBURG, Dietmar**
**D-3350 Kreiensen 1 (DE)**
• **NÄGERL, Hans**
**D-3407 Gleichen/OT Klein-Lengden (DE)**

(74) Vertreter: **Zapf, Christoph, Dipl.-Ing.**
**Patentanwälte Dr. Solf und Zapf**
**Postfach 13 01 13**
**42028 Wuppertal (DE)**

(56) Entgegenhaltungen:
EP-A- 0 442 330     EP-A- 0 498 586
WO-A-87/04917     DE-A- 3 908 958
GB-A- 2 199 753     US-A- 3 748 662
US-A- 4 224 696

# Beschreibung

Die vorliegende Erfindung betrifft ein künstliches Gelenk, insbesondere eine Endoprothese für das menschliche Kniegelenk, bestehend aus einem ersten, aus einem ersten Gelenkkopf und einer ersten Gelenkpfanne gebildeten Gelenk und einem zweiten, aus einem zweiten Gelenkkopf und einer zweiten Gelenkpfanne gebildeten Gelenk, die zueinander parallel angeordnet sind, wobei die jeweiligen Rotationsachsen der beiden Gelenkteile parallel zueinander verlaufen.

Aus der WO-A-90/11062 entsprechend der deutschen Patentanmeldung P 39 08 958.4 ist bereits ein künstliches Gelenk bekannt, zum Ersatz insbesondere von menschlichen Gelenken, bestehend aus mindestens zwei Gelenkteilen mit zueinander sich bewegenden sphärischen Funktionsflächen. Die Krümmungsverhältnisse der eine kreisförmige Schnittkontur aufweisenden Funktionsflächen sind zueinander konvex-konvex, konvex-konkav oder konkav-konkav, und die Gelenkgeometrie ist durch eine Gelenkkette mit zwei Gelenkachsen (dimere Gelenkkette) bestimmt, die durch die Rotationszentren der Funktionsflächen verlaufen. Hierbei sind die Gelenkflächen kugelförmig ausgebildet, so daß eine Gelenkbewegung mit fünf Freiheitsgraden möglich ist.

Aus der US-A 3,748,662 ist ein künstliches Kniegelenk der eingangs beschriebenen Art bekannt, von denen das eine Gelenkteil als ein kugelgelenkartiges Gelenk und das andere derart ausgebildet ist, daß es einen wulstförmigen Gelenkkopf aufweist, der in einer angepaßten, rinnenförmigen Pfanne gelagert ist und die laterale Kondyle in posteriorer Richtung überragt. Somit weist dieses Gelenk sowohl in der Sagitalebene als auch in der Frontalebene unterschiedliche, kreisförmige Schnittkonturen auf. Hierbei liegen die Gelenkachsen in der Längsebene gesehen in der gleichen Ebene. Die Krümmungsverhältnisse in den beiden Einzelgelenken sind jeweils konvex-konkav.

Aus der EP-A-2 0 442 330 ist ein Kniegelenk der eingangs beschriebenen Art bekannt, bei der die Gelenkachsen ebenfalls in derselben Ebene liegen und wobei ein Gelenkteil als Kugelgelenk ausgebildet ist und das andere Gelenkteil eine zylindrische Lagerfläche aufweist.

Es hat sich gezeigt, daß derartige Gelenke nicht geeignet sind, um die speziellen Gelenkfunktionen des menschlichen Kniegelenks exakt nachzubilden.

Der Erfindung liegt die Aufgabe zugrunde, ein künstliches Gelenk zu schaffen, das eine Bewegungsfreiheit nur in einer Gelenkebene besitzt und das gleichzeitig eine hohe mechanische Stabilität mit einer großen Variationsbreite zur Anpassung an individuelle Gegebenheiten aufweist.

Erfindungsgemäß wird dies durch ein künstliches Gelenk erreicht, das aus einem ersten, aus einem ersten Gelenkkopf und einer ersten Gelenkpfanne gebildeten Gelenk und einem zweiten, aus einem zweiten Gelenkkopf und einer zweiten Gelenkpfanne gebildeten Gelenk besteht, die zueinander parallel angeordnet sind, so daß die geweiligen Rotationsachsen der beiden Gelenke parallel zueinander verlaufen, und daß das zweite Gelenk gegenüber dem ersten Gelenk in der Längsebene gesehen zurückversetzt ist sowie die Gelenkköpfe untereinander und die Gelenkpfannen untereinander starr verbunden sind und die Gelenkköpfe und die Gelenkpfannen Gelenkflächen besitzen, die Flächensegmente eines toroidförmigen Körpers sind und die in zueinander senkrechten Ebenen, einer Längsebene und einer Querebene, Funktionsflächen besitzen, die in jeder einzelnen Ebene eine einzige kreisförmige Schnittkontur mit konstantem Radius aufweisen, wobei der Radius der kreisförmigen Schnittkontur in den beiden Ebenen unterschiedlich ist und wobei die Krümmungsverhältnisse der Funktionsflächen in jeder der Ebenen entweder konvex-konvex, konvex-konkav oder konkav-konkav sind, und die Gelenkgeometrie der Funktionsflächen zueinander in jeder der beiden Ebenen durch eine Gelenkkette mit zwei Gelenkachsen (dimere Gelenkkette) bestimmt ist, die durch die Rotationszentren der Funktionsflächen mit den jeweiligen Radien der jeweils zugehörigen Schnittkonturen verlaufen. Somit wird erfindungsgemäß ein als Viergelenk ausgebildetes künstliches Kniegelenk geschaffen, das derart konstruiert ist, daß unter der Wirkung der kraftschlüssigen kompressiven Kräfte der Unterschenkel nur nach hinten schwenken kann.

Hierbei ist es weiterhin vorteilhaft, wenn ein Druckverteilungskörper zwischen den Gelenkkörpern angeordnet ist, dessen an den Gelenkflächen anliegende Gleitflächen eine den Gelenkflächen angepaßte toroidförmige Ausgestaltung aufweisen, wobei der Druckverteilungskörper eine minimale Dicke besitzt, die auf der Verbindungslinie der Rotationszentren der die kreisförmigen Schnittkonturen aufweisenden Funktionsflächen liegt. Durch die erfindungsgemäß vorgesehenen toroidförmigen Gelenkflächen ergibt sich eine Bewegungsfreiheit nur in einer der Ebenen, und zwar vorzugsweise in der Längsebene, wohingegen in der hierzu senkrechten Querebene eine eingeschränkte Bewegungsfreiheit vorhanden ist. Die Erfindung beruht auf der überraschenden Erkenntnis, daß die Gelenkbahnen des menschlichen Kniegelenks durch jeweils toroidförmige Flächen der erfindungsgemäßen Ausbildung der Schnittkonturen in den zueinander senkrechten Ebenen ersetzt werden können. Die hierbei auftretenden Druckbeanspruchungen können durch die Verwendung entsprechend fester Materialien beherrscht werden. Der vorteilhafterweise vorhandene Druckverteilungskörper gewährleistet eine druckkraftschlüssige Bindung zwischen den beiden Gelenkteilen und eine Verteilung der auftretenden Druckbelastungen auf eine größere Fläche, woraus sich eine hohe mechanische Stabilität ergibt und ermöglicht wird, Materialien zu verwenden, die eine geringere mechanische Druckfestigkeit besitzen.

Es ist weiterhin vorteilhaft, wenn die die kreisbogenförmigen Schnittkonturen aufweisenden Funktionsflächen beider Ebenen, der Längs- und der Querebene, konvex-konkav derart ausgebildet sind, daß ihre Rotationszentren innerhalb des Gelenkteils, insbesondere einem ersten Gelenkkopf mit der konvexen kreisförmigen Schnittkontur liegen und die Gelenkachsenbahn der Rotationszentren einen Radius $R_M = R_2 - R_1 - D$ bzw. einen Abstand $R_{M1} = R_{22} - R_{11} - D$ besitzt, wobei $R_2$ größer ist als die Summe $R_1$ und D bzw. $R_{22}$ größer/gleich ist als die Summe aus $R_{11}$ und D, und die Rotationzentren der Funktionsflächen der Querebene nicht mit den Rotationsachsen durch die Rotationszentren der Funktionsflächen der Längsebene zusammenfallen. Hierbei ist für die Ausführungsform ohne Druckverteilungskörper D=0. Die Rotationszentren $M_{11}$ und $M_{22}$ müssen nicht zusammenfallen. Das Rotationszentrum $M_{22}$ kann in Richtung des Oberschenkels und/oder seitlich nach innen (medial) oder außen (lateral) versetzt von dem Rotationszentrum $M_{11}$ liegen. Dieses derart ausgestaltete Gelenk eignet sich insbesondere als Endoprothese für den medialen Gelenkteil des menschlichen Kniegelenks. Dieses mediale Gelenkteil soll die natürliche Artikulation zwischen dem medialen Oberschenkel (Femur) und medialen Schienbein (Tibia) Gelenkkopf ersetzen. Hierbei wird in der sagittalen Ebene eine überschlagene druckkraftschlüssige dimere Gelenkkette gebildet, die wegen ihrer toroidförmigen Ausformung der Gelenkflächen eine eingeschränkte Bewegungsfreiheit in der frontalen Ebene, das ist die senkrecht zur sagittalen Ebene verlaufende Ebene, aufweist.

Weiterhin kann es zweckmäßig sein, wenn die die kreisbogenförmigen Schnittkonturen aufweisenden Funktionsflächen derartige Krümmungsverhältnisse in der einen Ebene - der Längsebene - besitzen, daß sie konvex-konvex derart ausgebildet sind, daß ihre Rotationszentren in dem jeweils zugehörigen Gelenkteil liegen und die Gelenkachsenbahn der Rotationszentren einen Radius $R_L = R_8 + R_9 + D$ besitzt sowie die in der anderen Ebene - der Querebene - liegenden Funktionsflächen Krümmungsverhältnisse ihrer kreisförmigen Schnittkonturen mit den Radien $R_{81}$, $R_{91}$ derart besitzen, daß sie konvex-konkav ausgebildet sind, so daß ihre Rotationszentren $M_{81}$, $M_{91}$ innerhalb des Gelenkteils mit der konvexen Schnittkontur der Funktionsfläche liegen und die Rotationszentren einen Abstand $R_{L1} = R_{91} - R_{81} - D$ besitzen, wobei $R_{91}$ größer/gleich ist wie die Summe aus $R_{81}$ und der minimalen Dicke D des Druckverteilungskörpers sowie die Rotationszentren $M_{81}$ und $M_{91}$ nicht mit den Rotationsachsen durch die Rotationszentren $M_8$ und $M_9$ zusammenfallen. Auch hierbei gilt für die Ausgestaltung ohne Druckverteilungskörper die Bedingung D=0. Die Rotationszentren $M_{81}$ und $M_{91}$ müssen ebenfalls nicht zusammenfallen. Das Rotationszentrum $M_{91}$ kann in Richtung auf den Oberschenkel und/oder seitlich nach innen (medial) oder außen (lateral) versetzt von dem Rotationszentrum $M_{81}$ liegen. Dieses derart ausgebildete Gelenk stellt zweckmäßigerweise die Endoprothese für das laterale Gelenkteil des menschlichen Kniegelenks dar, das die natürliche Artikulation zwischen dem lateralen, femoralen (Oberschenkel) und lateralen tibialen (Schienbein) Kondylus ersetzen soll. Hierbei weist dieses Gelenk in der sagittalen Ebene (Längsebene) eine nicht überschlagene druckkraftschlüssige dimere Gelenkkette auf, die wegen ihrer toroidförmigen Ausformung der Gelenkflächen eine eingeschränkte Bewegungsfreiheit in der frontalen Ebene (Querebene) besitzt.

Weitere vorteilhafte Eigenschaften sind in den weiteren Unteransprüchen enthalten.

Anhand der in den beiliegenden Zeichnungen enthaltenen Ausführungsbeispiele wird die Erfindung näher erläutert. Es zeigen:

Fig. 1      einen Schnitt in der sagittalen Ebene (Längsebene) durch eine erste Ausführungsform eines erfindungsgemäßen Gelenks mit Druckverteilungskörper,

Fig. 2      einen Schnitt durch das Gelenkteil gemäß Fig. 1, jedoch in der frontalen Ebene, d. h. der um 90° versetzten Querebene zu Fig. 1,

Fig. 3      einen Schnitt in der sagittalen Ebene (Längsebene) durch eine weitere Ausführungsform eines erfindungsgemäßen Gelenkes mit Druckverteilungskörper,

Fig. 4      einen Schnitt durch das Gelenk gemäß Fig. 3, jedoch in der frontalen Ebene, d. h. in einer um 90° gedrehten Querebene zu Fig. 3,

Fig. 5      eine Anordnung eines rechten menschlichen Knies im sagittalen Schnitt (Längsschnitt, seitliche Ansicht).

Fig.6 u 7      Ansichten gemäß den Fig. 1 und 2 durch ein erfindungsgemäßes Gelenk ohne Druckverteilungskörper,

Fig.8 u 9      Ansichten gemäß den Fig. 3 und 4 durch ein erfindungsgemäßes Gelenk ohne Druckverteilungskörper,

Fig. 10      eine Anordnung eines erfindungsgemäßen rechten menschlichen Knies im sagittalen Schnitt in einer weiteren Ausführungsform.

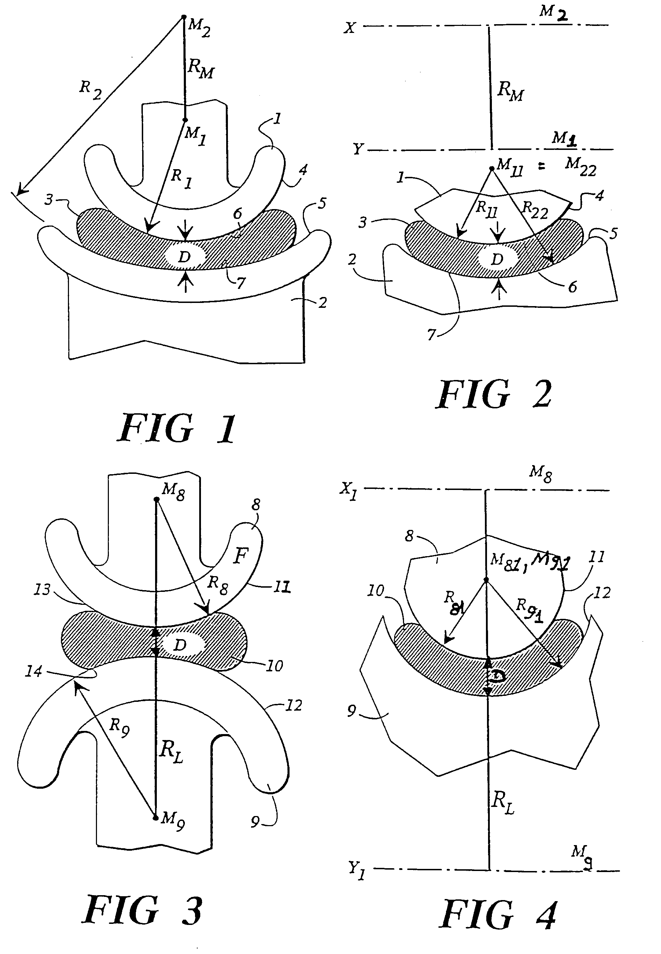

Ein menschliches Kniegelenk besteht aus zwei Gelenkteilen, und zwar dem medialen Gelenkteil und dem lateralen Gelenkteil. In Fig. 1 ist der Schnitt durch das erfindungsgemäße künstliche Gelenk dargestellt, das als Ersatz für das mediale Gelenkteil dienen kann. Dieses erfindungsgemäße Gelenk soll die natürliche Artikulation zwischen dem medialen femoralen (Oberschenkel) Gelenkkopf (Kondylus) und dem medialen tibialen (Schienbein) Gelenkkopf (Kondylus) ersetzen. Dieses erste, erfindungsgemäße Gelenk besteht aus zwei Gelenkteilen 1 und 2, wobei das Gelenkteil 1, erster Gelenkkopf, dem Femurteil (Oberschenkelteil) entsprechen kann, das starr mit dem Knochen des medialen femoralen Kondylus verbunden wird. Das Gelenkteil 2, erste Gelenkpfanne, entspricht dem Tibiateil (Schienbeinteil), der starr mit dem Knochen des tibialen Kondylus verbunden wird. Zwischen den beiden Gelenkteilen 1 und 2 befindet sich ein Druckverteilungskörper 3. Gemäß der vorliegenden Erfindung ist nun vorgesehen, daß die Gelenkfläche 4 des Gelenkteils 1 und die Gelenkfläche 5 des Gelenkteils 2 jeweils toroidförmig ausgebildet sind. Die den Gelenkflächen 4, 5 zugekehrten Gleitflächen 6, 7 des Diskus 3 sind entsprechend in Anpassung an die Gelenkflächen 4, 5 toroidförmig ausgestaltet. Wie sich aus Fig. 1 ergibt, weist das Gelenkteil 1 in der sagittalen Schnittebene, d. h. in der Längsebene, eine Funktionsfläche mit kreisbogenförmiger Schnittkontur auf, deren Rotationszentrum $M_1$ ist und wobei die kreisförmige Schnittkontur den Radius $R_1$ besitzt. Hierbei besitzt die derart gebildete Funktionsfläche eine konvexe Ausbildung. Das Gelenkteil 2 besitzt ebenfalls eine kreisförmige Schnittkontur mit dem Rotationszentrum $M_2$ und mit dem Radius $R_2$, so daß sich eine konkave Ausbildung der durch die kreisförmige Schnittkontur gebildeten Funktionsfläche ergibt. Hierbei ist eine derartige Anordnung gegeben, daß diese Rotationszentren $M_1$ und $M_2$ innerhalb des Gelenkteils mit der konvexen Schnittkontur liegen und die Gelenkachsenbahn der Rotationszentren einen Radius $R_M = R_2 - R_1 - D$ besitzen. Hierbei ist D die minimale Dicke des Diskus 3 auf der Verlängerung der Verbindungslinie der beiden Rotationszentren $M_1$ und $M_2$. Hierbei ist $R_2$ derart bemessen, daß die Summe aus $R_1$ und D kleiner ist als $R_2$. Somit stellt diese Anordnung eine überschlagene dimere Gelenkkette dar.

In Fig. 2 ist zu erkennen, daß auch in der Querebene, d. h. der Frontalebene, die Funktionsflächen der beiden Gelenkteile 1, 2 kreisförmige Schnittkonturen besitzen, wobei die kreisförmige Schnittkontur der Funktionsfläche des Gelenkteils 1 den Radius $R_{11}$ und den Mittelpunkt bzw. das Rotationszentrum $M_{11}$ besitzt und am Gelenkteil 2 die Funktionsfläche den Radius $R_{22}$ sowie den Mittelpunkt $M_{22}$. Auch hier ist wiederum der Druckverteilungskörper 3 zwischen den beiden Gelenkteilen 1, 2 zu erkennen mit seiner minimalen Diskusdicke D. Im dargestellten Ausführungsbeispiel fallen die Mittelpunkte $M_{11}$ und $M_{22}$ aufeinander. Die Rotationszentren $M_{11}$ und $M_{22}$ müssen nicht zusammenfallen. Das Rotationszentrum $M_{22}$ kann in Richtung des Oberschenkels und/oder seitlich nach innen (medial) oder außen (lateral) versetzt von dem Rotationszentrum $M_{11}$ liegen. Dies ist jedoch lediglich eine zweckmäßige Ausgestaltung. Weiterhin ist in Fig. 2 zu erkennen, wie die Rotationsachsen X, Y durch die Rotationszentren $M_2$ und $M_1$ in Fig. 1 innerhalb des frontalen Schnittes gemäß Fig. 2 verlaufen. Dabei ist zu erkennen, daß die

Mittelpunkte $M_{11}$ und $M_{22}$ nicht mit den Rotationsachsen X, Y durch die Mittelpunkte $M_1$ bzw. $M_2$ zusammenfallen.

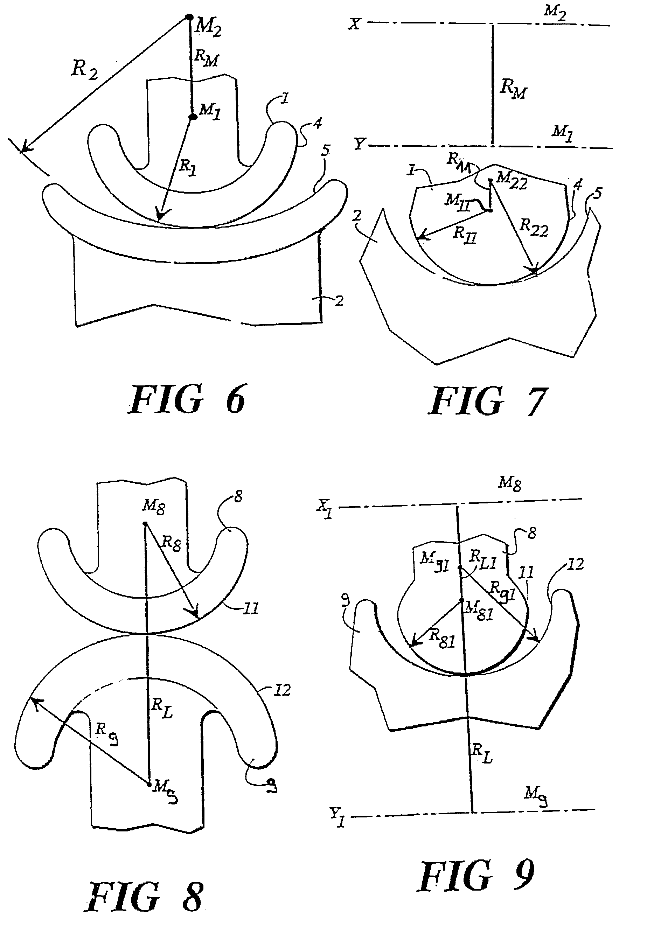

In den Fig. 6 und 7 ist ein dem Gelenk gemäß Fig. 1 und 2 entsprechendes Gelenk gemäß der Erfindung jedoch ohne Druckverteilungskörper dargestellt. Hierbei sind gleiche Teile jeweils mit denselben Bezugsziffern versehen. In bezug auf die Dimensionierung der geometrischen Beziehungen der jeweiligen Radien zueinander gilt die Bedingung D=0. Geht man dabei davon aus, daß der Radius $R_M$ bei beiden Gelenken gemäß Fig. 1 und 2 bzw. Fig. 6 und 7 gleich groß ist, so wird man zweckmäßigerweise beim Gelenk gemäß den Fig. 6, 7 $R_1$ und $R_{11}$ gegenüber den entsprechenden Radien in den Fig. 1, 2 vergrößern. Diese Maßnahme ist deshalb zweck-mäßig, da hierdurch die Berührungsfläche zwischen den Gelenkflächen 5 und 6 möglichst groß ist.

In Fig. 3 ist wiederum ein Schnitt durch die Längsebene bzw. in der sagittalen Ebene eines zweiten, erfindungsgemäßen Gelenks dargestellt, das den lateralen Gelenkteil eines menschlichen Kniegelenkes bilden kann. Der laterale Gelenkteil ersetzt die natürliche Artikulation zwischen dem lateralen femoralen und lateralen tibialen Kondylus. Das Gelenk gemäß Fig. 3 besteht aus den Gelenkteilen 8, 9, und zwar einem zweiten Gelenkkopf 8 und einer zweiten Gelenkpfanne 9, zwischen denen ein Druckverteilungskörper 10 beweglich angeordnet ist. Das Gelenkteil 8 stellt dabei das Femurteil dar, das starr mit dem Knochen des lateralen femoralen Kondylus verbunden wird im menschlichen Körper, und das Gelenkteil 9 stellt das Tibiateil dar, das starr mit dem Knochen des lateralen tibialen Kondylus im menschlichen Körper verbunden wird. Die von den Gelenkteilen 8, 9 gebildeten Gelenkflächen 11, 12 sind toroidförmig ausgebildet, so daß die den Gelenkflächen 11, 12 zugekehrten Gleitflächen 13, 14 des Diskus 10 ebenfalls in Anpassung an die Ausgestaltung der Gelenkflächen 11, 12 toroidförmig ausgestaltet sind. Das Gelenkteil 8 weist in Fig. 3 in seiner Längsebene ebenfalls eine Funktionsfläche mit einer kreisförmigen Schnittkontur auf, wobei diese kreisförmige Schnittkontur den Mittelpunkt bzw. das Rotationszentrum $M_8$ und den Radius $R_8$ besitzt, so daß sich ein konvexer Verlauf der Funktionsfläche ergibt. Das Gelenkteil 9 weist den Mittelpunkt $M_9$ bzw. das Rotationszentrum $M_9$ auf und besitzt den Radius $R_9$, so daß sich ebenfalls eine konvexe Form der kreisbogenförmigen Schnittkontur der Funktionsfläche ergibt. Wie dargestellt ist, liegen die Rotationszentren $M_8$ und $M_9$ jeweils im zugehörigen Gelenkteil 8 und 9. Die Gelenkachsenbahn der Rotationszentren $M_8$ und $M_9$ besitzt einen Radius $R_L = R_8 + R_9 + D$, wobei D die minimale Dicke des Diskus 10 auf der Verbindung zwischen den beiden Rotationszentren $M_8$ und $M_9$ ist.

In Fig. 4 ist der Schnitt gemäß der Frontalebene, Querebene, zu der Darstellung in Fig. 3 dargestellt. Hierbei ist zu erkennen, daß auch in dieser Schnittebene die Gelenkkörper 8, 9 jeweils kreisförmige Schnittkonturen ihrer Funktionsflächen besitzen. Der Gelenkkörper 8 weist dabei eine kreisförmige Schnittkontur mit dem Mittelpunkt $M_{81}$ und dem Radius $R_{81}$ auf, wobei eine konvexe Funktionsfläche gebildet wird. Der Gelenkkörper 9 besitzt den Mittelpunkt $M_{91}$ und den Radius $R_{91}$, wobei eine konkave Ausbildung der kreisförmigen Schnittkontur besteht. Hierbei liegen die Rotationszentren bzw. die Mittelpunkte $M_{81}$ und $M_{91}$ innerhalb des Gelenkteils 8 mit der konvexen Schnittkontur der Funktionsfläche, und die Gelenkachsenbahn der Rotationszentren $M_{81}$, $M_{91}$ ist im dargestellten Ausführungsbeispiel aufeinanderfallend angeordnet. Die Rotationszentren $M_{81}$ und $M_{91}$ müssen ebenfalls nicht zusammenfallen. Das Rotationszentrum $M_{91}$ kann in Richtung auf den Oberschenkel und/oder seitlich nach innen (medial) oder außen (lateral) versetzt von dem Rotationszentrum $M_{81}$ liegen. Weiterhin ist zu erkennen, daß die Rotationszentren $M_{81}$ und $M_{91}$ nicht mit den Rotationsachsen $X_1$, $Y_1$ durch die Rotationszentren $M_8$ und $M_9$ zusammenfallen. Die kreisbogenförmigen Funktionsflächen in Fig. 3 stellen eine nicht überschlagene druckkraftschlüssige dimere Gelenkkette dar, wobei wegen der toroidförmigen Ausformung der Gelenkflächen keine Bewegungsfreiheit in der frontalen Ebene, siehe Fig. 4, gegeben ist.

Wesentlich für die einwandfreie Funktion der erfindungsgemäßen Gelenke gemäß den Fig. 1 bis 4 ist, daß die Druckverteilungskörper 3 bzw. 10 beweglich sind. Hierzu müßten die Gleitflächen 6, 7 zwischen den Druckverteilungskörpern und den Gelenkteilen 1, 2 bzw. 8, 9 eine möglichst geringe Reibung zu den Gelenkteilen besitzen, und zudem muß die Reibungskraft zwischen den anliegenden Flächen beidseitig des Druckverteilungskörpers jeweils gleich groß sein. Dies wird dadurch erreicht, daß die wirksamen Gleitflächen 6, 7 der Druckverteilungskörper 3, 10 zu den jeweils angrenzenden Gelenkteilen, das sind die Gelenkteile 1, 2 bzw. 8, 9, gleich groß ausgelegt werden. Die Größe der jeweiligen Kontaktfläche kann durch die Wahl der toroidalen Wulstradien sowie durch die Gestaltung der Randwülste der Druckverteilungskörper erreicht werden. Zudem sind die Gleitflächen 6, 7; 13, 14 hochglanzpoliert.

In den Fig. 8 und 9 ist ein dem Gelenk gemäß den Fig. 3 und 4 entsprechendes Gelenk jedoch ohne Druckverteilungskörper dargestellt. Hierbei sind gleiche Teile jeweils mit denselben Bezugsziffern versehen. Hinsichtlich der geometrischen Beziehungen der jeweiligen Radien zueinander gilt hier die Bedingung D=0. Geht man hierbei davon aus, daß der Radius $R_L$ bei beiden Gelenken gemäß den Fig. 3, 4 und den Fig. 8, 9 gleich groß ist, so wird man zweckmäßigerweise $R_8$ und $R_{81}$ gemäß den Fig. 6 und 7 größer wählen als beim Gelenk gemäß Fig. 3, 4. Durch diese Maßnahme wird die Berührungsfläche zwischen den Gelenkflächen 11, 12 möglichst groß.

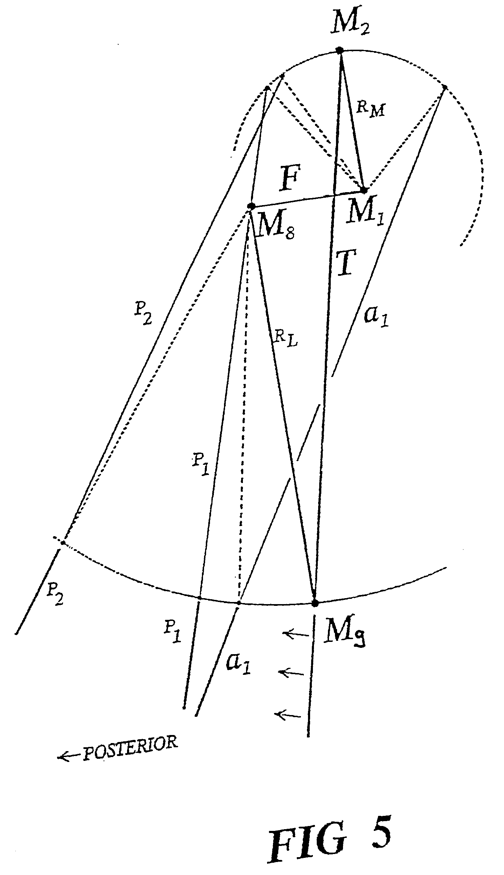

In Fig. 5 ist schematisch der Aufbau eines rechten Knies im sagittalen Schnitt mit seitlicher Ansicht dargestellt, und zwar mittels des ersten Gelenks der Fig. 1, 2 bzw. Fig. 6, 7 und des zweiten Gelenks gemäß Fig. 3, 4 bzw. Fig. 8, 9. Hierbei sind die einzelnen Gelenkteile derart miteinander gekoppelt, daß der femorale erste und zweite Gelenkkopf 1, 8 und die tibiale erste und zweite Gelenkpfanne 2, 9 jeweils starr miteinander verbunden sind. Die beiden Gelenke aus den Gelenkteilen 1, 2 bzw. 8, 9 sind erfindungsgemäß so eingebaut, daß ihre vier Drehachsen X, Y und $X_1$ und $Y_1$ durch die Rotationszentren $M_1$ und $M_2$ bzw. $M_8$ und $M_9$ parallel zueinander in zwei parallelen Ebenen verlaufen und daß das zweite Gelenk aus den Gelenkteilen 8, 9 gegenüber dem ersten Gelenk aus den Gelenkteilen 1, 2 etwas nach posterior, d. h. nach hinten, versetzt ist. Ein derartiges Gelenkgebilde stellt ein Viergelenk dar, wobei das in Fig. 5 mit F gekennzeichnete Gelenkteil mit dem Femur, und das mit T dargestellte Gelenkteil mit der Tibia verbunden ist. Im Koordinatensystem des Femur ist F das Gestell, T die Koppel, $R_L$ und $R_M$ die rotierenden Glieder. Die Relativbewegung der Tibia gegenüber dem Femur stellt sich also als Bewegung der Koppel T dar. Da die Länge von T größer ist als die Summe aus $R_M + R_L$ kann sich die Achse $M_8$ aus der dick gezeichneten Initialstellung heraus nur nach posterior bewegen. Die Achse $M_2$ kann sich sowohl nach anterior als nach posterior bewegen. In beiden Fällen schwenkt aber die distale Verlängerung von T, der Unterschenkel, nach hinten. Beide Fälle stellen zwei mögliche Kniebeugebewegungen dar, wobei jede einzelne für sich zwangläufig abläuft. Bei der anterioren Bewegung der Drehachse $M_2$ bewegt sich diese nach Überschreiten der anterioren Totlage (RM und T bilden eine gerade Linie und fallen zusammen) weiter nach anterior. Die Tibia kann dann die dünn gezeichnete Stellung $a_1$ einnehmen. Bei der posterioren Bewegung von $M_2$ erreicht diese Achse in der posterioren Totlage ($R_L$ und T bilden eine gerade Linie und fallen zusammen, gezeichnete Lage $p_1$) ihre posteriorste Lage. Im Gefolge der weiteren Bewegung wandert $M_2$ dann in anteriore Richtung. Diese Wanderung geschieht so langsam, daß bei diesem weiteren Schwenken des Unterschenkels (Tibia T) die Drehachse $M_2$ an ihrem Ort zu verharren scheint (Lage $p_2$ der Tibia). In jedem Fall ($a_1$, $p_1$, $p_2$) ist der Unterschenkel nach hinten geschwenkt. Das künstliche Gelenk ist also so konstruiert, daß unter der Wirkung der kraftschlüssigen kompressiven Kräfte der Unterschenkel nur nach hinten schwenken kann.

Weiterhin ist es möglich, die minimale Stärke der Druckverteilungskörper auf Null zu reduzieren, so daß sich ein ringförmiger Ring-Druckverteilungskörper mit einer mittleren Öffnung ergibt.

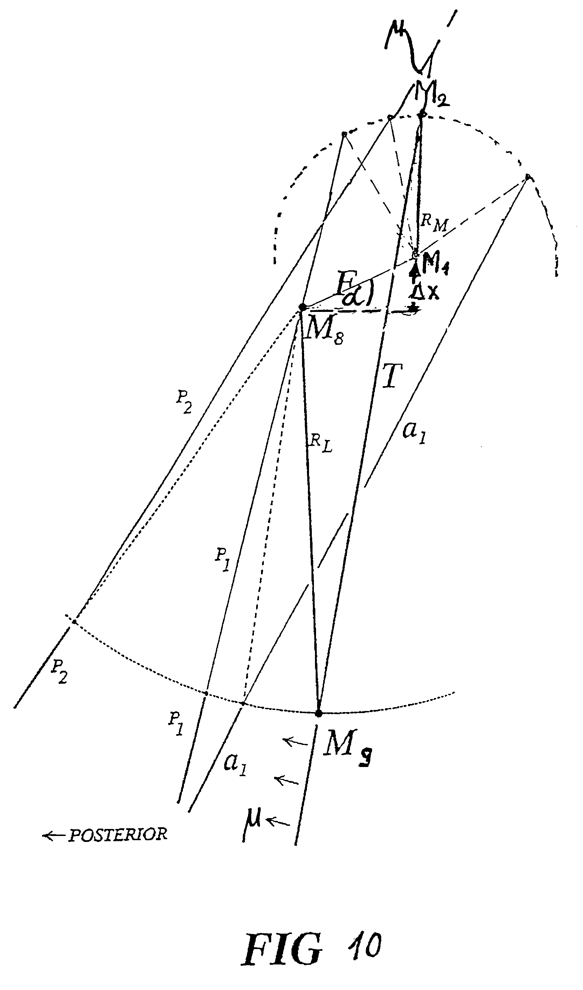

In Fig. 10 ist eine weitere Ausgestaltung eines erfindungsgemäßen Gelenks in der Darstellung gemäß Fig. 5 gezeigt. Diese Gelenkanordnung zum Ersatz des menschlichen Kniegelenks zeigt, daß das erste Gelenk aus den Gelenkteilen 1, 2, das medial angeordnet ist, gegenüber dem lateral angeordneten Gelenk aus den Gelenkteilen 8, 9 in Richtung auf den Femur hinsichtlich seiner Gelenkmittelpunkte $M_1$, $M_2$ um das Maß $\Delta x$ ver-

setzt ist. Durch die Größe dieses Versatzes kann der maximale Schwingwinkel $\mu_{max}$ des Gelenks, der dem maximalen Beugewinkel des menschlichen Knies entspricht, nach posterior beeinflußt werden. Hierbei besteht die Gesetzmässigkeit, daß je größer der femurale Versatz $\Delta x$ ist, umso kleiner der maximale Schwingwinkel $\mu_{max}$ ausfällt. Vorzugsweise wird das femurale Versatzmaß $\Delta x$ derart gewählt, daß die Verbindungsstrecke F von $M_8$ nach $M_1$ gegenüber der Horizontallinie einen Winkel $\alpha$ einschließt, der zwischen 0 und 45° liegt: $0 < \alpha < 45°$. Auch liegt es im Rahmen der vorliegenden Erfindung, die Gelenkteile 1, 2 bzw. 8, 9 des erfindungsgemäßen Gelenks derart anzuordnen, daß die Ebenen durch die Koppelglieder $R_L$ und $R_M$ räumlich zueinander geneigt verlaufen, woraus sich ein sphärischer Bewegungsablauf ergibt.

## Patentansprüche

1. Künstliches Gelenk, insbesondere Endoprothese für das menschliche Kniegelenk, bestehend aus einem ersten, aus einem ersten Gelenkkopf (1) und einer ersten Gelenkpfanne (2) gebildeten Gelenk und einem zweiten, aus einem zweiten Gelenkkopf (8) und einer zweiten Gelenkpfanne (9) gebildeten Gelenk, die zueinander derart parallel angeordnet sind, daß die jeweiligen Rotationsachsen (X, Y,/X1, Y1) der beiden Gelenke parallel zueinander verlaufen, und daß das zweite Gelenk gegenüber dem ersten Gelenk in der Längsebene gesehen zurückversetzt ist sowie die Gelenkköpfe (1, 8) untereinander und die Gelenkpfannen (2, 9) untereinander starr verbunden sind, und die Gelenkköpfe (1, 8) und die Gelenkpfannen (2, 9) Gelenkflächen besitzen, die Flächensegmente eines toroidförmigen Körpers sind und die in zueinander senkrechten Ebenen, einer Längsebene und einer Querebene, Funktionsflächen besitzen, die in jeder einzelnen Ebene eine einzige kreisförmige Schnittkontur mit konstantem Radius aufweisen, wobei der Radius der kreisförmigen Schnittkontur in den beiden Ebenen unterschiedlich ist und wobei die Krümmungsverhältnisse der Funktionsflächen in jeder der Ebenen entweder konvex-konvex, konvex-konkav oder konkav-konkav sind, und die Gelenkgeometrie der Funktionsflächen zueinander in jeder der beiden Ebenen durch eine Gelenkkette mit zwei Gelenkachsen bestimmt ist, die durch die Rotationszentren ($M_1$, $M_2$, $M_{11}$, $M_{22}$; $M_8$, $M_9$, $M_{81}$, $M_{91}$) der Funktionsflächen mit den Radien ($R_1$, $R_2$, $R_{11}$, $R_{22}$; $R_8$, $R_9$, $R_{81}$, $R_{91}$) der jeweils zugehörigen Schnittkonturen verlaufen.

2. Gelenk nach Anspruch 1,
   **gekennzeichnet durch** einen Druckverteilungskörper (3, 10) zwischen den Gelenkflächen (4, 5; 11, 12), dessen an den Gelenkflächen anliegende Gleitflächen (6, 7; 13, 14) eine den Gelenkflächen angepaßte toroidförmige Ausgestaltung aufweisen, wobei der Druckverteilungskörper (3, 10) eine minimale Dicke besitzt, die auf der Verbindungslinie der Rotationszentren der die kreisförmige Schnittkonturen aufweisenden Funktionsflächen liegt.

3. Gelenk nach Anspruch 1 oder 2,
   **dadurch gekennzeichnet,** daß die die kreisförmigen Schnittkonturen aufweisenden Funktionsflächen der Gelenkteile (1, 2) des ersten Gelenks in beiden Ebenen, in der Längsebene und der Querebene, konvex-konkav derart ausgebildet sind, daß ihre Rotationszentren ($M_1$, $M_2$) innerhalb des Gelenkteils (1), dem Gelenkkopf, mit der konvexen kreisförmigen Schnittkontur liegen und die Gelenkachsenbahn der Rotationszentren einen Radius $R_M = R_2 - R_1$ bzw. einen Abstand $R_{M1} = R_{22} - R_{11}$ ohne Druckverteilungskörper besitzt, oder $R_M = R_2 - R_1 - D$ bzw. einen Abstand $R_{M1} = R_{22} - R_{11} - D$ mit Druckverteilungskörper, wobei $R_2$ bzw. $R_{22}$ größer/gleich ist wie die Summe aus $R_1$ bzw. $R_{11}$ und die minimale Dicke D des Druckverteilungskörpers, wobei $D = 0$ ist für den Fall ohne Druckverteilungskörper, und die Rotationszentren ($M_{11}$, $M_{22}$) der Funktionsflächen der Querebene nicht mit den Rotationsachsen (X, Y) durch die Rotationszentren ($M_1$, $M_2$) der Funktionsflächen der Längsebene zusammenfallen.

4. Gelenk nach Anspruch 3,
   **dadurch gekennzeichnet,** daß die Rotationszentren $M_{11}$ und $M_{22}$ zusammenfallen oder voneinander derart abweichen, daß das Rotationszentrum $M_{22}$ in Richtung des Oberschenkels und/oder seitlich nach innen (medial) oder nach außen (lateral) versetzt von dem Rotationszentrum $M_{11}$ liegt.

5. Gelenk nach Anspruch 3 oder 4,
   **dadurch gekennzeichnet,** daß die konvexen Funktionsflächen an einem ersten Gelenkkopf (1) und die konkaven Funktionsflächen an einer ersten Gelenkpfanne (2) ausgebildet sind.

6. Gelenk nach einem der Ansprüche 1 bis 5,
   **dadurch gekennzeichnet,** daß das die kreisförmigen Schnittkonturen der Funktionsflächen der Gelenkteile (8, 9) des zweiten Gelenks mit den Radien ($R_8$, $R_9$) in der einen Ebene - der Längsebene - konvex-konvex derart ausgebildet sind, daß ihre Rotationszentren ($M_8$, $M_9$) in dem jeweils zugehörigen Gelenkteil (8, 9) liegen und die Gelenkachsenbahn der Rotationszentren ($M_8$, $M_9$) einen Radius $R_L = R_8 + R_9$ ohne Druckverteilungskörper und $R_L = R_8 + R_9 + D$ mit Druckverteilungskörper besitzt sowie in der anderen Ebene - der Querebene - die Krümmungsverhältnisse der kreisförmigen Schnittkonturen mit den Radien $R_{81}$, $R_{91}$ derart konvex-konkav ausgebildet sind, daß ihre Rotationszentren ($M_{81}$, $M_{91}$) innerhalb des Gelenktelis (8) mit der konvexen Schnittkontur der Funktionsfläche liegen und der Abstand der Rotationszentren $R_{L1} = R_{91} - R_{81}$ ohne Druckverteilungskörper und $R_{L1} = R_{91} - R_{81} - D$ mit Druckverteilungskörper ist, wobei $R_{91}$ größer/gleich ist wie die Summe aus $R_{81} + D$, der minimalen Dicke des Druckverteilungskörpers, wobei $D = 0$ ist für den Fall ohne Druckverteilungskörper sowie die Rotationszentren ($M_{81}$, $M_{91}$) nicht mit den Rotationsachsen ($X_1$, $Y_1$) durch die Rotationszentren ($M_8$, $M_9$) zusammenfallen.

7. Gelenk nach Anspruch 6,
**dadurch gekennzeichnet**, daß die Rotationszentren $M_{81}$ und $M_{91}$ zusammenfallen oder derart auseinanderliegen, daß das Rotationszentrum $M_{91}$ in Richtung auf den Oberschenkel und/oder seitlich nach innen (medial) oder nach außen (lateral) versetzt von dem Rotationszentrum $M_{81}$ liegt.

8. Gelenk nach Anspruch 6 oder 7,
**dadurch gekennzeichnet,** daß an einem zweiten Gelenkkopf (8) die Funktionsflächen der beiden Ebenen konvex und an einer zweiten Gelenkpfanne (9) die Funktionsflächen in der Längsebene konvex und in der Querebene konkav ausgebildet sind.

9. Gelenk nach einem der Ansprüche 1 bis 8,
**dadurch gekennzeichnet,** daß die beiden Gleitflächen (5, 6; 13, 14) der Druckverteilungskörper (3, 10) gleich groß sind.

**Claims**

1. An artificial joint, in particular an endoprosthesis for the human knee joint, comprising a first joint formed from a first joint head (1) and first joint socket (2), and a second joint formed from a second joint head (8) and a second joint socket (9), which are arranged parallel to one another such that the respective axes of rotation (X, Y,/X1, Y1) of the two joints run parallel to one another, and such that the second joint is set back with respect to the first joint, as seen in the longitudinal plane, and the joint heads (1, 8) are rigidly connected to one another and the joint sockets (2, 9) are rigidly connected to one another, and the joint heads (1, 8) and the joint sockets (2, 9) have joint faces which are face segments of a toroidal body and have functional faces in mutually perpendicular planes, a longitudinal plane and a transverse plane, these functional planes having a single circular sectional contour of constant radius in each individual plane, the radius of the circular sectional contour being different in the two planes and the relationships between the curvatures of the functional faces in each of the planes either being convex-convex, convex-concave or concave-concave, and the joint geometry of the functional faces with respect to one another in each of the two planes being determined by a joint chain having two joint axes which run through the centres of rotation ($M_1$, $M_2$, $M_{11}$, $M_{22}$; $M_8$, $M_9$, $M_{81}$, $M_{91}$) of the functional faces with the radii ($R_1$, $R_2$, $R_{11}$, $R_{22}$; $R_8$, $R_9$, $R_{81}$, $R_{91}$) of the respectively associated sectional contours.

2. A joint according to Claim 1, characterized by a pressure distribution body (3, 10) between the joint faces (4, 5; 11, 12), whereof sliding faces (6, 7; 13, 14) bearing against the joint faces have a toroidal shape matched to the joint faces, the pressure distribution body (3,10) having a minimal thickness which lies on the line connecting the centres of rotation of the functional faces having the circular sectional contours.

3. A joint according to Claim 1 or 2, characterized in that the functional faces of the joint parts (1, 2) of the first joint, having the circular sectional contours, are constructed in both planes, in the longitudinal plane and the transverse plane, to be convex-concave such that their centres of rotation ($M_1$, $M_2$) lie within the joint part (1), the joint head having the convex circular sectional contour, and the path of the joint axes of the centres of rotation has a radius $R_M = R_2 - R_1$ or a spacing $R_{M1} = R_{22} - R_{11}$ without the pressure distribution body, or $R_M = R_2 - R_1 - D$ or a spacing $R_{M1} = R_{22} - R_{11} - D$ with the pressure distribution body, where $R_2$ or $R_{22}$ is greater than or equal to the total of $R_1$ or $R_{11}$ and the minimum thickness D of the pressure distribution body, where $D = 0$ for the case without a pressure distribution body, and the centres of rotation ($M_{11}$, $M_{22}$) of the functional faces of the transverse plane do not coincide with the axes of rotation (X, Y) through the centres of rotation ($M_1$, $M_2$) of the functional faces of the longitudinal plane.

4. A joint according to Claim 3, characterized in that the centres of rotation $M_{11}$ and $M_{22}$ coincide or deviate from one another such that the centre of rotation $M_{22}$ lies offset from the centre of rotation $M_{11}$ in the direction of the thigh and/or laterally inwards (medial) or outwards (lateral).

5. A joint according to Claim 3 or 4, characterized in that the convex functional faces are constructed on a first joint head (1) and the concave functional faces are constructed on a first joint socket (2).

6. A joint according to one of Claims 1 to 5, characterized in that the circular sectional contours of the functional faces of the joint parts (8, 9) of the second joint having the radii ($R_8$, $R_9$) are constructed in the one plane - the longitudinal plane - to be convex-convex such that their centres of rotation ($M_8$, $M_9$) lie in the respectively associated joint part (8, 9), and the path of the joint axes of the centres of

rotation ($M_8$, $M_9$) has a radius $R_L = R_8 + R_9$ without the pressure distribution body and $R_L = R_8 + R_9 + D$ with the pressure distribution body, and in the other plane - the transverse plane - the ratios of curvature of the circular sectional contours having the radii $R_{81}$, $R_{91}$ are constructed to be convex-concave such that their centres of rotation ($M_{81}$, $M_{91}$) lie within the joint part (8) having the convex sectional contour of the functional face and the spacing between the centres of rotation is $R_{L1} = R_{91} - R_{81}$ without the pressure distribution body and $R_{L1} = R_{91} - R_{81} - D$ with the pressure distribution body, where $R_{91}$ is greater than or equal to the total of $R_{81} + D$, the minimum thickness of the pressure distribution body, where D = 0 for the case without the pressure distribution body and the centres of rotation ($M_{81}$, $M_{91}$) do not coincide with the axes of rotation ($X_1$, $Y_1$) through the centres of rotation ($M_8$, $M_9$).

7. A joint according to Claim 6, characterized in that the centres of rotation $M_{81}$ and $M_{91}$ coincide or lie apart such that the centre of rotation $M_{91}$ lies offset from the centre of rotation $M_{81}$ in the direction of the thigh and/or laterally inwards (medial) or outwards (lateral).

8. A joint according to Claim 6 or 7, characterized in that on a second joint head (8) the functional faces of the two planes are constructed to be convex and on a second joint socket (9) the functional faces are constructed to be convex in the longitudinal plane and concave in the transverse plane.

9. A joint according to one of Claims 1 to 8, characterized in that the two sliding faces (5, 6; 13, 14) of the pressure distribution bodies (3, 10) are the same size.

**Revendications**

1. Articulation artificielle, en particulier endoprothèse pour l'articulation du genou humain, consistant en une première articulation comportant une première tête d'articulation (1) et une première cavité articulaire (2), et une deuxième articulation comportant une deuxième tête d'articulation (8) et une deuxième cavité articulaire (9), ces articulations étant disposées parallèlement entre elles de manière à ce que les axes de rotation respectives (X, Y ; X1, Y1) des deux articulations s'étendent parallèlement entre eux, et que la deuxième articulation soit disposée en retrait par rapport à la première articulation lorsqu'on regarde dans le plan longitudinal, les têtes d'articulation (1, 8) et les cavités articulaires (2, 9) étant respectivement reliées entre elles de manière rigide, les têtes d'articulation (1, 8) et les cavités articulaires (2, 9) présentant des surfaces d'articulation qui sont des segments de surface d'un corps toroïdal et sont munies, dans des plans perpendiculaires entre eux, à savoir un plan longitudinal et un plan transversal, de surfaces fonctionnelles qui présentent dans chacun des plans un seul contour de coupe circulaire à rayon constant, le rayon du contour de coupe circulaire étant différent dans les deux plans, la courbure des surfaces fonctionnelles dans chaque plan étant soit convexe-convexe, soit convexe-concave, soit concave-concave, la géométrie d'articulation des surfaces fonctionnelles entre elles dans chacun des deux plans étant déterminée par une chaîne d'articulation comportant deux axes d'articulation qui s'étendent à travers les centres de rotation ($M_1$, $M_2$, $M_{11}$, $M_{22}$ ;, $M_8$, $M_9$, $M_{81}$, $M_{91}$) des surfaces fonctionnelles de rayons ($R_1$, $R_2$, $R_{11}$, $R_{22}$ ; $R_8$, $R_9$, $R_{81}$, $R_{91}$) des contours de coupe respectivement associés.

2. Articulation selon la revendication 1, caractérisée par un corps de répartition de la pression (3, 10) disposé entre les surfaces d'articulation (4, 5 ; 11, 12) dont les surfaces de glissement (6, 7 ; 13, 14) appliquées aux surfaces d'articulation ont une forme toroïdale adaptée aux surfaces d'articulation, le corps de répartition de la pression (3, 10) ayant une épaisseur minimum située sur la ligne de connexion des centres de rotation des surfaces fonctionnelles présentant les contours de coupe circulaires.

3. Articulation selon la revendication 1 ou 2, caractérisée en ce que les surfaces fonctionnelles présentant les contours de coupe circulaires des éléments d'articulation (1, 2) de la première articulation ont, dans les deux plans, à savoir le plan longitudinal et le plan transversal, une forme convexe-concave telle que leurs centres de rotation ($M_1$, $M_2$) soient situés à l'intérieur de l'élément d'articulation (1), à savoir la tête d'articulation, ayant le contour de coupe circulaire convexe, et que le trajet de l'axe d'articulation des centres de rotation présente un rayon $R_M = R_2 - R_1$, respectivement une distance $R_{M1} = R_{22} - R_{11}$ sans corps de répartition de la pression, ou $R_M = R_2 - R_1 - D$, respectivement une distance $R_{M1} = R_{22} - R_{11} - D$ avec le corps de répartition de la pression, $R_2$, respectivement $R_{22}$ étant supérieur ou égal à la somme de $R_1$, respectivement $R_{11}$ et de l'épaisseur minimum D du corps de répartition de la pression, D = 0 dans le cas sans corps de répartition de la pression, et que les centres de rotation ($M_{11}$, $M_{22}$) des surfaces fonctionnelles du plan transversal ne coïncident pas avec les axes de rotation (X, Y) passant par les centres de rotation ($M_1$, $M_2$) des surfaces fonctionnelles du plan longitudinal.

4. Articulation selon la revendication 3, caractérisée en ce que les centres de rotation ($M_{11}$)

et $(M_{22})$ coincident ou différent entre eux de manière à ce que le centre de rotation $(M_{22})$ soit décalé dans la direction de la cuisse et/ou latéralement vers l'intérieur (décalage médial) ou l'extérieur (décalage latéral) par rapport au centre de rotation $(M_{11})$.

5. Articulation selon la revendication 3 ou 4, caractérisée en ce que les surfaces fonctionnelles convexes sont formées sur une première tête d'articulation (1) et les surfaces fonctionnelles concaves sur une première cavité articulaire (2).

6. Articulation selon l'une des revendications 1 à 5, caractérisée en ce que les contours de coupe circulaires des surfaces fonctionnelles des éléments d'articulation (8, 9) de la deuxième articulation qui ont des rayons $(R_8, R_9)$ ont dans un plan - le plan longitudinal - une forme convexe-convexe telle que leurs centres de rotation $(M_8, M_9)$ soient situés dans l'élément d'articulation respectivement associé (8, 9), et que le trajet de l'axe d'articulation des centres de rotation $(M_8, M_9)$ présente un rayon $R_L = R_8 + R_9$ sans corps de répartition de la pression, et $R_L = R_8 + R_9 + D$ avec le corps de répartition de la pression, la courbure du contour de coupe circulaire avec les rayons $(R_{81})$ et $(R_{91})$ étant dans l'autre plan - le plan transversal - convexe-concave de manière à ce ses centres de rotation $(M_{81}, M_{91})$ soient situés dans l'élément d'articulation (8) dont les surfaces fonctionnelles ont un contour de coupe convexe, et que la distance entre les centres de rotation est de $R_{L1} = R_{91} - R_{81}$ sans corps de répartition de la pression, et de $R_{L1} = R_{91} - R_{81} - D$ avec un corps de répartition de la pression, $R_{91}$ étant supérieur ou égal à la somme de $R_{81} + D$, l'épaisseur minimum du corps de répartition de la pression, $D = 0$ dans le cas sans corps de répartition de la pression, les centres de rotation $(M_{81}, M_{91})$ ne coïncidant pas avec les axes de rotation $(X_1, Y_1)$ passant par les centres de rotation $(M_8, M_9)$.

7. Articulation selon la revendication 6, caractérisée en ce que les centres de rotation $(M_{81})$ et $(M_{91})$ coïncident ou sont espacés de manière à ce que le centre de rotation $(M_{91})$ soit décalé dans la direction de la cuisse et/ou latéralement vers l'intérieur (décalage médial) ou l'extérieur (décalage latéral) par rapport au centre de rotation $(M_{81})$.

8. Articulation selon la revendication 6 ou 7, caractérisée en ce que sur une deuxième tête d'articulation (8), les surfaces fonctionnelles des deux plans ont une forme convexe, et sur une deuxième cavité articulaire (9), les surfaces fonctionnelles ont une forme convexe dans le plan longitudinal et concave dans la plan transversal.

9. Articulation selon l'une des revendications 1 à 8, caractérisée en ce que les deux surfaces de glissement (5, 6 ; 13, 14) des corps de répartition de la pression sont de mêmes dimensions.

FIG 1

FIG 2

FIG 3

FIG 4

FIG 5

**FIG 6**

**FIG 7**

**FIG 8**

**FIG 9**

**FIG 10**